# EUROPEAN PATENT APPLICATION

(11) **EP 3 699 341 A1**
(43) Date of publication of application: **26.08.2020**
(21) Application number: 18868321.3
(22) Date of filing: 17.05.2018
(51) Int. Cl.: D03D 15/02

(54) **ANTI-ELECTROMAGNETIC-POLLUTION CONNECTABLE FABRIC**

(30) Priority: 19.10.2017 ES 201731253 U
(71) Applicant: Representaciones De Descanso, S.L., 03830 Muro De Alcoy (Alicante) (ES)
(72) Inventor: BERENGUER JOVER, Oscar, 03830 Muro De Alcoy (Alicante) (ES)
(74) Representative: Munoz Garcia, Antonio
(86) International application number: PCT/ES2018/070360
(87) International publication number: WO 2019/077175

(57) **Abstract**

The present invention relates to a connectable anti-electromagnetic-pollution textile, configured from a piece of fabric (2) made with fibers comprising, in variable proportions, metal fibers (3), and a discharge device (4) with means for connecting said fabric (2), via a connector (5) provided therein for said purpose, to an earth connection via a conventional outlet. Optionally, the fabric (2) includes tourmaline-based composite additives incorporated during production, and, preferably, the discharge device (4) comprises a conducting cable (6) having a snap-type connector terminal (7) at one end for connection to the connector (5) provided at a point of the fabric (2), and a jack-type connector terminal (8) at the opposite end for connection to an interchangeable plug (9) for a conventional power outlet. The cable (6) is at least partially extendable.

## Description

### OBJECT OF THE INVENTION

As expressed in the title of the present specification, the invention relates to a connectable anti-electromagnetic-pollution textile which provides the intended function thereof with advantages and features that are described in detail below and constitute a novelty in the current state of the art.

More specifically, the object of the invention focuses on a textile, applicable for making different elements, preferably bed or home linens, such as upholstery or textiles for the production of mattresses, whose constitution and connectability, by means of a discharge device, to an earth connection via a conventional outlet, is particularly conceived for mitigating electromagnetic pollution existing in many environments today, which textile is furthermore also optionally prepared for reducing stress caused by excessive positive ions of users in contact with it.

### FIELD OF APPLICATION OF THE INVENTION

The field of application of the present invention is comprised in the sector of the textile industry, focusing particularly on the field of making textiles and fabrics for making home or household linens, as well as for making elements intended for rest, covering at the same time the field of systems and devices intended for eliminating or mitigating electromagnetic waves.

### BACKGROUND OF THE INVENTION

As is known, there are increasingly more elements which generate artificial electromagnetic radiation due to the exponential increase of elements ranging from electric lines, wireless mobile telephony, to home appliances around homes and work or school or recreational activity centers where people anywhere worldwide today spend time.

Said radiation, as recognized by the WHO (World Health Organization) itself, affects health as it can cause thermal and athermal effects that can lead to biological changes, as well as to a whole series of symptoms suffered particularly by people with electrosensitivity, estimated to possibly reach up to 10% of the population, such as headaches, insomnia, irritability, depression, or even a higher cancer risk.

Moreover, it is also known that in many people stress is often caused by excessive positive ions in the environment or environments in which they spend time. In addition to the natural sources of positive ions which are damaging to human beings, such as for example, the atmosphere hours before a storm, the wind coming from dry areas, or others, most positive ion accumulation comes from artificial sources such as: artificial radioactivity, atmospheric pollution, air conditioning, synthetic fibers, computer screens, DVD screens, and television screens (cathode-ray tube). In contrast, a place filled with negative ions prevents said type of stress and provides many benefits.

The objective of the present invention therefore is to develop means for alleviating and reducing the mentioned effects of electromagnetic radiation and of positive ions through the textile used in elements intended for rest or for the household with which users' bodies are in contact.

Moreover and in reference to the current state of the art, it must be pointed out that the applicant at least is unaware of the existence of any other connectable textile or similar invention having technical, structural, and constitutive features that are identical or similar to those herein claimed.

### DISCLOSURE OF THE INVENTION

The connectable anti-electromagnetic-pollution textile proposed by the invention is therefore configured as a remarkable novelty in its field of application, given that the objectives indicated above are specifically and satisfactorily achieved in view of its implementation, with the characterizing details making it possible and distinguishing same being suitably described in the final claims accompanying the present description.

Specifically, as indicated above, the invention proposes a textile, applicable for making different elements, such as bed or home linens, mattresses, pillows, or other elements intended for rest or for the household, whose constitution and configuration give it the capacity to be connected to an earth connection via a conventional outlet, and to passively neutralize the electromagnetic radiation from the immediate surroundings once connected, and thereby mitigating and reducing pollution and harmful effects which said radiation may eventually cause to the health of the people who are sensitive to same, and which textile, in an alternative embodiment option thereof, is furthermore prepared to actively cause the release of negative ions for the purpose of counteracting an excessive charge of positive ions, and thereby reducing the stress produced by said cause in users who are in contact with the fabric.

To that end and more specifically, the connectable textile proposed by the invention is essentially configured from a fabric with fibers comprising metal fibers, and a discharge device which allows connecting said fabric to an earth connection via a conventional outlet.

Therefore, once the textile is connected with the discharge device, both elements act passively, i.e., without user intervention, capturing the electromagnetic radiation pollution present in the environment surrounding the part made with said textile, naturally shunting it to the earth connection of the outlet, without using any type of power supply.

Furthermore, in a more complete embodiment option, the textile of the invention, in addition to the metal fibers with which the fabric thereof is made, includes tourmaline-based composite additives incorporated therein during manufacture, which additives, as is known, consist of a mineral made up of aluminum silicate with sodium, boron, magnesium, iron, and other minerals which produce different varieties, as a result of which the textile reacts with the body movements of a user who is with contact with it, for example, in an element intended for rest such as a pillow, while at the same time interacting with their body heat, causing the fabric to release beneficial negative ions with these two processes, reducing the number of harmful, stress-generating cations.

Finally, it must be pointed out that, in the preferred embodiment, the discharge device is configured from a conducting cable having a female snap-type connector terminal at one end, suitable for connection to the male snap connector provided for that purpose at least at one point of the fabric by pressure, and a male or female jack-type connector terminal at the opposite end which is in turn sized for connection to an independent plug, which can be interchanged to use the one that best suits the needs of each place, to enable plugging into the conventional power outlet of any home, since, as is known, said outlets vary from country to country, and to thereby allow discharging electromagnetic radiation via the current outlet to which all the current outlets of a conventional installation are connected.

Furthermore, the cable is preferably at least partially extendable by means of winding same into a coil, to allow connecting the textile to an outlet located a certain distance from same.

The described connectable anti-electromagnetic-pollution textile therefore represents an innovation in structural and constitutive features that have been unknown up until now, and these reasons, combined with its practical usefulness, provide it with sufficient grounds to obtain the exclusive privilege that is sought.

### DESCRIPTION OF THE DRAWINGS

To complement the description that is being made and for the purpose of helping to better understand the features of the invention, a set of drawings is attached to the present specification as an integral part thereof, in which the following is depicted with an illustrative and non-limiting character:
Figure 1 shows a schematic perspective view of an embodiment of the connectable anti-electromagnetic-pollution textile of the invention, with the main parts and elements it comprises, specifically those of the textile and those of the discharge device, which have been depicted separately, as well as the configuration and arrangement thereof, being shown.
Figure 2 shows a perspective view of the discharge device the invention comprises, depicted in a disassembled position, with all the parts and elements it comprises being shown.

### PREFERRED EMBODIMENT OF THE INVENTION

In view of the mentioned figures and according to the numbering used, there can be observed therein a non-limiting embodiment of the connectable anti-electromagnetic-pollution textile of the invention, which comprises parts and elements that are indicated and described in detail below.

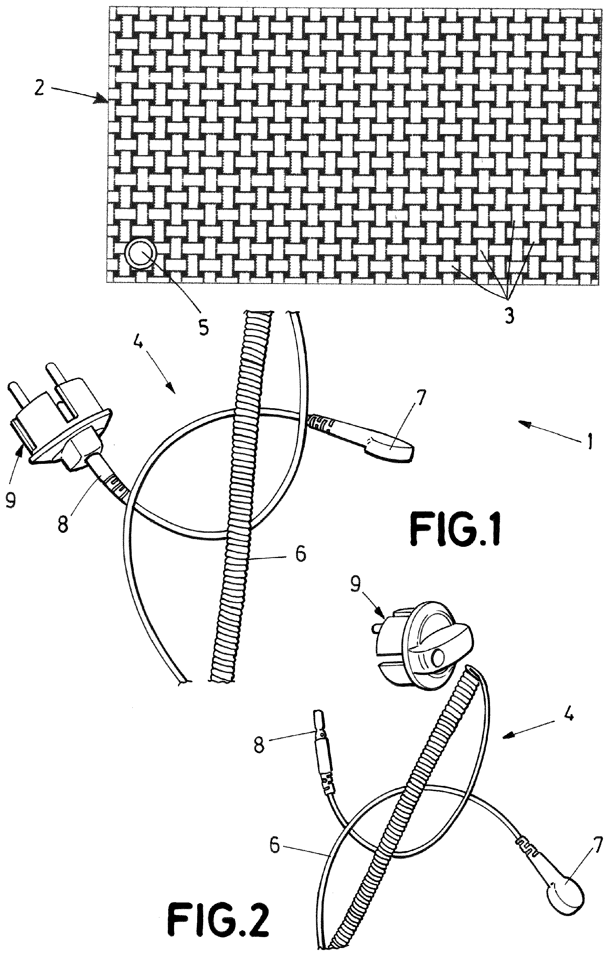

In this sense, as observed in Figure 1, the connectable textile (1) in question is essentially configured from a piece of fabric (2) which can have any size, configuration, and shape, depending on the element for which it is intended, said fabric being made with fibers comprising, in variable proportions, metal fibers (3), and a discharge device (4) with means for connecting said fabric (2), via a connector (5) provided therein for said purpose, to an earth connection via a conventional outlet (not depicted), such that the electromagnetic radiation of the immediate surroundings of said fabric (2) is neutralized by connecting said fabric (2) to the earth connection by means of the discharge device (4).

Furthermore, the fabric (2) optionally includes tourmaline-based composite additives incorporated during production, also in a variable proportion, in any case sufficient for it to react with the body movements of a user who is in contact with the fabric (2) and with their body heat, causing the release of negative ions.

In the preferred embodiment, the discharge device (4) is configured from a conducting cable (6) having a male or female snap-type connector terminal (7) at one end, suitable for connection to the connector (5) of the fabric (2) by pressure, consisting of a complementary male snap connector provided at least at one point of the fabric (2); and a male or female jack-type connector terminal (8) at the opposite end of said cable (6) which is in turn sized for connection to an interchangeable plug (9), suitable for being able to be plugged into the conventional power outlet of any home, which allows using the one that best suits the needs of each place or country.

Preferably, the cable (6) is at least partially extendable by means of winding a section thereof into a coil.

Having sufficiently described the nature of the present invention, as well as the manner of putting it into practice, it is not considered necessary to expand on this explanation so that one skilled in the art will understand its scope and the advantages derived from it, hereby stating that within its essential nature, the invention may be carried out to practice in other embodiments which differ in detail from that indicated by way of example and which will likewise attain the protection that is sought provided that its fundamental principle is not altered, changed, or modified.

## Claims

1. A connectable anti-electromagnetic-pollution textile, **characterized by** being configured from a piece of fabric (2) made with fibers comprising, in variable proportions, metal fibers (3), and a discharge device (4) with means for connecting said fabric (2), via a connector (5) provided therein for said purpose, to an earth connection via a conventional outlet.

2. The connectable anti-electromagnetic-pollution textile according to claim 1, **characterized in that** the fabric (2) includes tourmaline-based composite additives incorporated during production.

3. The connectable anti-electromagnetic-pollution textile according to claim 1 or 2, **characterized in that** the discharge device (4) is configured from a conducting cable (6) having a snap-type connector terminal (7) at one end, suitable for connection to the connector (5) of the fabric (2) by pressure, consisting of a complementary snap connector provided at least at one point of the fabric (2); and a jack-type connector terminal (8) at the opposite end of said cable (6) which is in turn sized for connection to an interchangeable plug (9), suitable for being able to be plugged into a conventional power outlet.

4. The connectable anti-electromagnetic-pollution textile according to claim 3, **characterized in that** the cable (6) is at least partially extendable.
